# EUROPÄISCHE PATENTANMELDUNG

(11) **EP 1 878 386 A1**
(43) Veröffentlichungstag der Anmeldung: **16.01.2008**
(21) Anmeldenummer: 06014792.3
(22) Anmeldetag: 15.07.2006
(51) Int. Cl.: A61B 5/15, A61B 19/12

(54) **Verfahren zum Herstellen einer Lanzette, Lanzette, Lanzettenvorratsband und Stechgerät zum Erzeugen einer Einstichwunde mittels einer solchen Lanzette**

(71) Anmelder: Roche Diagnostics GmbH, 68305 Mannheim (DE); F.HOFFMANN-LA ROCHE AG, 4070 Basel (CH)
(72) Erfinder: Macho, Heinz, 64658 Fürth (DE); Roeper, Josef, 67141 Neuhofen (DE)
(74) Vertreter: Mommer, Niels

(57) **Zusammenfassung**

Die Erfindung betrifft eine Verfahren zum Herstellen einer Lanzette, bei dem ein Lanzettenkörper einschließlich einer Lanzettenspitze mittels Laserschneiden aus einem Metallband ausgeschnitten wird sowie eine auf diese Weise hergestellte Lanzette. Die Erfindung betrifft ferner ein Lanzettenvorratsband mit mehren Lanzetten, die jeweils in Kammern (12) einer Folienverpackung (11) versiegelt sind, wobei die Kammern (12) der Folienverpackung (11) ein Band bilden. Die Erfindung betrifft ferner ein Stechgerät zum Erzeugen einer Einstichwunde mittels einer austauschbaren Lanzette (10), umfassend einen Lanzettenantrieb (21) zum Bewegen einer in das Stechgerät (20) eingesetzten Lanzette (10) für einen Einstich, wobei der Lanzettenantrieb (21) für einen Einstich ein Drehmoment auf eine eingesetzte Lanzette (10) überträgt, so dass die eingesetzte Lanzette (10) eine Einstichbewegung in Form einer Drehbewegung ausführt.

## Beschreibung

Die Erfindung betrifft ein Verfahren zum Herstellen einer Lanzette, die einen Lanzettenkörper mit einer Lanzettenspitze umfasst, wobei der Lanzettenkörper mitsamt der Lanzettenspitze aus einem Metallband ausgeschnitten wird, sowie eine Lanzette mit einem Lanzettenkörper aus Metallblech, ein Lanzettenvorratsband und ein Stechgerät zum Erzeugen einer Einstichwunde mittels austauschbarer Lanzetten.

Stechgeräte und dazugehörende Lanzetten werden beispielsweise von Diabetikern benötigt, die mehrmals täglich ihren Blutzuckerspiegel kontrollieren müssen, indem in einem Körperteil, in der Regel einem Finger, eine kleine Einstichwunde erzeugt wird, um eine Körperflüssigkeitsprobe zu gewinnen, die mit einem Messgerät zur Bestimmung des Glukosegehalts untersucht werden kann. Um die Bestimmung des Glukosegehalts für Diabetiker möglichst einfach zu gestalten, sind beispielsweise aus der WO 2004/086970 A1 Lanzetten bekannt, die mit einem Testfeld zur Untersuchung einer durch eine Einstichwunde gewonnenen Körperflüssigkeitsprobe ausgestattet sind.

Lanzetten werden als Verbrauchsgegenstände in großer Zahl benötigt und verursachen einen erheblichen Teil der mit der Behandlung von Diabetes verbundenen Kosten. Eine weit verbreitete Möglichkeit zur kostengünstigen Herstellung von Lanzetten besteht darin, aus einem Metallband einen Lanzettenkörper einschließlich einer Lanzettenspitze auszustanzen, wie es in der WO 2004/086970 A1 beschrieben ist. Zur Herstellung von Lanzetten aus Metallblech wird ferner in der EP 1346686 A2 der Einsatz von Ätztechniken genannt. Als Alternative zu Metallblech wurden beispielsweise in der WO 2005/084530 auch nichtmetallische Materialen, beispielsweise Silizium und Keramikmaterialen für die Herstellung der Lanzetten vorgeschlagen.

Aufgabe der Erfindung ist es, einen Weg aufzuzeigen, wie bei der Diabetesbehandlung die mit Lanzetten verbundenen Kosten weiter gesenkt werden können.

Diese Aufgabe wird erfindungsgemäß durch ein Verfahren zum Herstellen einer Lanzette gelöst, die einen Lanzettenkörper mit einer Lanzettenspitze umfasst, wobei der Lanzettenkörper mitsamt der Lanzettenspitze mittels eines Lasers aus einem Metallband ausgeschnitten wird. Diese Aufgabe wird ferner gelöst durch eine Lanzette umfassend einen mittels Laserschneiden aus einem Metallblech ausgeschnittenen Lanzettenkörper mit einer Lanzettenspitze.

Bei einem mittels Laserschneiden aus einem Metallband ausgeschnittenen Metallkörper finden sich an den Schnitträndern charakteristische Ablagerungen von Metallen und Metalloxiden, die durch den Laserstrahl aufgeschmolzen bzw. erzeugt wurden. Ausgestanzte Metallteile haben besonders scharfkantige Ränder, so dass eine durch Laserschneiden hergestellte Lanzette eindeutig von einer ausgestanzten Lanzette unterschieden werden kann.

Obwohl Lanzetten seit vielen Jahren in großen Stückzahlen hergestellt werden, können durch den erfindungsgemäßen Einsatz eines Lasers Einsparungspotentiale zur Kostensenkung ausgeschöpft werden. In anderen Bereichen der Technik ist das Laserschneiden für Kleinserien als Alternative zum Ausstanzen von Metallteilen gebräuchlich. Bei kleinen Stückzahlen ist das Laserschneiden in der Regel kostengünstiger als das Stanzen, da für die auszuschneidende Kontur kein individuell angefertigtes Stanzwerkzeug benötigt wird, dessen Kosten für eine wirtschaftliche Produktion auf große Stückzahlen umgelegt werden müssen. Es ist deshalb umso überraschender, dass sich trotz extrem hoher Stückzahlen Lanzetten mittels Laserschneiden kostengünstiger als durch Ausstanzen herstellen lassen.

Der Einsatz eines Lasers ermöglicht insbesondere bei der Herstellung von so genannten integrierten Lanzetten, die ein Testfeld zum Untersuchen einer durch eine Einstichwunde gewonnenen Körperflüssigkeitsprobe aufweisen, die Einsparung von Produktionskosten. Derartige Lanzetten mit einem metallischen Lanzettenkörper und einem Testfeld zu photometrischen oder elektrochemischen Konzentrationsbestimmung erfordern nach dem Stand der Technik eine Fülle von unterschiedlichen Fertigungstechnologien, um einerseits metallische Komponenten der Lanzette, das heißt den Lanzettenkörper und die Lanzettenspitze, und andererseits nichtmetallische Komponenten, insbesondere das Testfeld, zu bearbeiten. Diese unterschiedlichen Technologien müssen aufeinander abgestimmt werden so dass die Investitionskosten für eine Massenfertigung mit der Anzahl der eingesetzten Fertigungstechnologien stark anwachsen. Hinzu kommt, dass jeder Fertigungsschritt nur eine begrenzte Anlageneffizienz hat, so dass bei einer Fertigung, die mehrere Fertigungsschritte mit unterschiedlichen Technologien kombiniert, die Gefahr einer geringen Gesamtanlageneffizienz besteht.

Im Gegensatz zu den im Stand der Technik gebräuchlichen Fertigungstechnologien wie Ätzen oder Stanzen, lässt sich ein Laser nicht nur zur Bearbeitung metallischer Lanzettenkomponenten sondern auch für nichtmetallische Lanzettenkomponenten, wie beispielsweise einem Polymerfadengewebe mit Testchemikalien für eine in die Lanzette integrierte Testeinrichtung in Form eines Testfelds, nutzen. Mittels eines Lasers lassen sich der Lanzettenkörper samt Lanzettenspitze aus Metallblech ausschneiden, ein Polymerfadengewebe zuschneiden und dessen Ränder verschmelzen sowie eine Lanzette in eine Folienverpackung einschweißen.

Testfelder mit Testchemikalien, die durch eine Verfärbung die Konzentration eines Analyten, beispielsweise Glukose, anzeigen, sind sehr empfindlich. Lanzetten mit integrierten Testfeldern müssen deshalb bis zum Gebrauch trocken und vor Umwelteinflüssen geschützt gelagert werden. Dies lässt sich in vorteilhafter Weise dadurch realisieren, dass die Lanzetten einzeln in eine Folienverpackung aus Kunststoff eingeschweißt sind. Auch für diesen Herstellungsschritt ist ein Laser hervorragend geeignet.

Ein weiterer Aspekt der Erfindung, der auch eigenständige Bedeutung für auf andere Weise hergestellte Lanzetten haben kann, betrifft deshalb ein Lanzettenvorratsband, umfassend mehrere Lanzetten, die jeweils in Kammern einer Folienverpackung versiegelt sind, wobei die Kammern der Folienverpackung ein Band bilden. Ein derartiges Lanzettenvorratsband kann beispielsweise dadurch hergestellt werden, dass Lanzetten in einer Reihe auf einer Kunststofffolie angeordnet werden, anschließend mit einer zweiten Folie bedeckt werden und die aufeinander liegenden Folien rund um die Lanzetten jeweils miteinander verschweißt werden, so dass sich ein Band von luftdicht verschlossenen Kammern bildet.

Im Rahmen der Erfindung wurde festgestellt, dass sich flache, das heißt aus einem Metallband ausgeschnittene, Lanzetten aus einer Folienverpackung besonders leicht durch eine Drehbewegung entnehmen lassen. Anstelle der im Stand der Technik gebräuchlichen linearen Einstichbewegungen kann mit einer Lanzette auch durch eine Drehbewegung eine Einstichwunde zum Gewinnen einer Körperflüssigkeitsprobe erzeugt werden. Ein wichtiger Aspekt der Erfindung, der auch eigenständige Bedeutung haben kann, betrifft deshalb eine Lanzette mit einem Lanzettenkörper aus Metallblech, der zum Ankuppeln an einen Lanzettenantrieb eines Stechgeräts ein Kupplungselement aufweist, über das die Lanzette in eine Drehbewegung versetzbar ist. Die Erfindung betrifft ferner ein Stechgerät zum Erzeugen einer Einstichwunde mittels einer austauschbaren Lanzette umfassend einen Lanzettenantrieb zum Bewegen einer in das Gerät eingesetzten Lanzette für einen Einstich, dadurch gekennzeichnet, dass der Lanzettenantrieb für einen Einstich ein Drehmoment auf eine eingesetzte Lanzette überträgt, so dass diese eine Einstichbewegung in Form einer Drehbewegung ausführt. Es hat sicht gezeigt, dass es günstig ist, wenn die als Einstichbewegung ausgeführte bogenförmige Drehbewegung der Lanzettenspitze nicht mehr als 180°, bevorzugt nicht mehr als 90°, insbesondere nicht mehr als 45°, überstreicht.

Am einfachsten lässt sich das Kupplungselement als Ausnehmung in dem Lanzettenkörper ausbilden, die formschlüssig mit einem Lanzettenantrieb eines Stechgeräts kuppelbar ist, so dass ein Drehmoment von dem Lanzettenantrieb auf die Lanzette übertragen werden kann. Zum Kuppeln wird ein Antriebselement des Lanzettenantriebs, beispielsweise eine Welle mit einem zu der Ausnehmung passenden Querschnitt, in die Ausnehmung der Lanzette gesteckt, so dass sich eine Drehbewegung des Antriebselements auf die Lanzette überträgt. Prinzipiell ist hierfür jede von der Kreisform abweichende Kontur der Ausnehmung geeignet. Bevorzugt hat die Ausnehmung jedoch eine kantige, beispielsweise eine stern- oder vierkantförmige Kontur, da sich der Formschluss zwischen der Ausnehmung und dem Antriebselement des Lanzettenantriebs zum Austausch einer Lanzette in der Regel umso leichter lösen lässt, je deutlicher die Abweichung von der Kreisform ist.

Das Wort "formschlüssig" bedeutet in diesem Zusammenhang, dass eine Kraftübertragung zum Erzeugen einer Drehbewegung rechtwinklig zu den aneinander anliegenden Flächen der Verbindungspartner erfolgt. Die betreffenden Flächen sind einerseits die Außenfläche eines Verbindungselements, beispielsweise einer Welle, die an der Innenseite der Ausnehmung des Lanzettenkörpers, in der Regel einem durchgehenden Loch, anliegt. Da der Lanzettenkörper naturgemäß flach ist, ist die Innenfläche der Ausnehmung notwendigerweise sehr klein und kann im Extremfall auch durch eine Kante oder Linie gebildet werden, gegen die das Antriebselement zur Übertragung eines Drehmoments drückt.

Die Vorteile einer Lanzette mit einem Kupplungselement, über das die Lanzette in eine Drehbewegung versetzbar ist, sowie eines dazugehörenden Stechgeräts, bei dem der Lanzettenantrieb für einen Einstich ein Drehmoment auf eine eingesetzte Lanzette überträgt, so dass diese eine Einstichbewegung in Form einer Drehbewegung ausführt, lassen sich auch mit Lanzetten nutzen, deren Lanzettenkörper einschließlich der Lanzettenspitze aus einem Metallband ausgestanzt oder auf andere Weise hergestellt wurde. Lanzetten mit einem Lanzettenkörper, der zum Ankuppeln an einen Lanzettenantrieb eines Stechgeräts ein Kupplungselement aufweist, über das die Lanzette in eine Drehbewegung versetzbar ist, und das dazugehörende Stechgerät sind deshalb ebenso wie das angesprochene Lanzettenvorratsband mit einem Band von Kammern einer Folienverpackung, in denen sich jeweils eine Lanzette befindet, Aspekte der Erfindung mit eigenständiger Bedeutung.

Weitere Einzelheiten und Vorteile der Erfindung werden im Folgenden anhand von Ausführungsbeispielen unter Bezugnahme auf die beigefügten Zeichnungen erläutert. Gleiche oder entsprechende Teile sind dabei mit übereinstimmenden Bezugszeichen gekennzeichnet. Im Rahmen der Ausführungsbeispiele offenbarte Merkmale können einzeln oder in Kombination zum Gegenstand von Ansprüchen gemacht werden. Es zeigen:
- Fig. 1: ein Stahlblech, aus dem mittels eines Lasers ein Lanzettenkörper einschließlich einer Lanzettenspitze ausgeschnitten wird;
- Fig. 2: ein aus dem Metallband ausgeschnittenes Halbzeug, mit teilweise aus dem Band ausgeschnitten Lanzettenkörper;
- Fig. 3: das in Figur 2 dargestellte Halbzeug und einen daran befestigten Streifen mit Testchemikalien;
- Fig. 4: ein Ausführungsbeispiel fertiger Lanzetten;
- Fig. 5: ein Ausführungsbeispiel eines Lanzettenvorratsbands mit mehreren Lanzetten gemäß dem in Figur 4 dargestellten Ausführungsbeispiel, die jeweils in Kammern einer Folienverpackung versiegelt sind;
- Fig. 6: ein Ausführungsbeispiel eines Stechgeräts mit einem eingelegten Lanzettenvorratsband gemäß Figur 5;
- Fig. 7: ein weiteres Ausführungsbeispiel einer erfindungsgemäßen Lanzette in einer Folienverpackung;
- Fig. 8: das in Figur 7 dargestellte Ausführungsbeispiel nach Herausdrehen aus der Folienverpackung für eine Probenaufnahme;
- Fig. 9: das in den Figuren 7 und 8 dargestellte Ausführungsbeispiel nach Gebrauch in einer Messposition;
- Fig. 10: ein Ausschnitt eines Metallbands 1 mit Begrenzungsstreifen zwischen die zum Ausbilden eines Testfelds eine Flüssigkeit mit Testchemikalien aufgebracht wird;
- Fig. 11: eine Querschnittsansicht zu Figur 10;
- Fig. 12: das Metallband gemäß Figur 10 mit aufgebrachter Flüssigkeit;
- Fig. 13: eine Querschnittsansicht zu Figur 12;
- Fig. 14: das Metallband nach Eintrocknen der Flüssigkeit; und
- Fig. 15: eine Querschnittsansicht zu Figur 14.

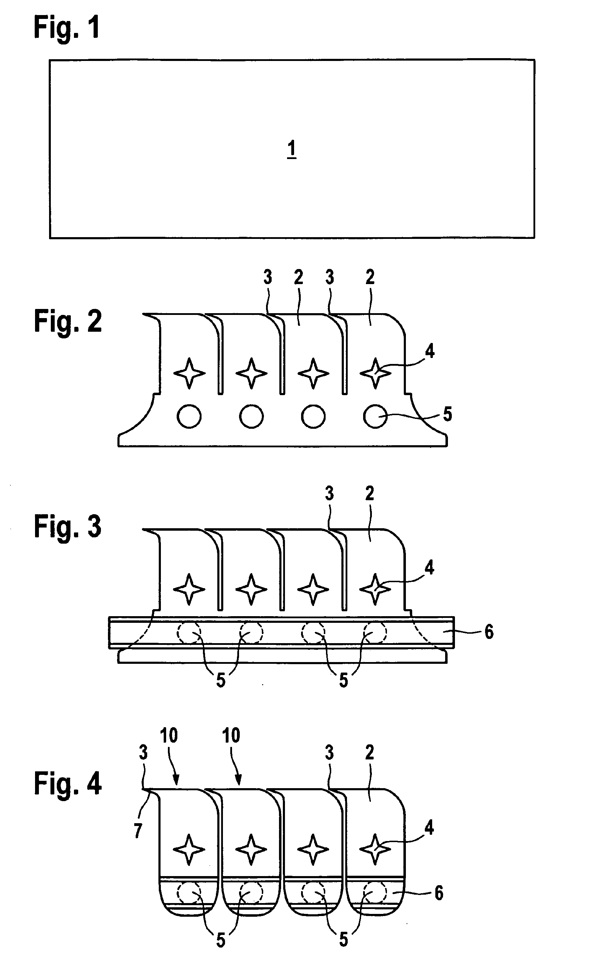

Im Folgenden wird anhand der Figuren 1 bis 4 ein Ausführungsbeispiel eines Verfahrens zur Herstellung eines Ausführungsbeispiels einer Flachlanzette erläutert. Aus dem in Figur 1 dargestellten Metallband 1 werden mittels Laserschneiden Lanzettenkörper 2 einschließlich Lanzettenspitzen 3 ausgeschnitten. Bei den dargestellten Ausführungsbeispielen ist das Metallband 1 ein Stahlblech, dessen Stärke 50 µm bis 200 µm, bevorzugt 60 µm bis 100 µm, beträgt. Für das Laserschneiden kann beispielsweise ein Nd:YAG-Laser verwendet werden.

Beim Schneiden des Metallbands 1 ist es günstig, das Metallband 1 relativ zu dem Laserstrahl zu bewegen, obwohl es prinzipiell selbstverständlich auch möglich ist, den Fokus des Laserstrahls über die Oberfläche des Metallbands 1 zu bewegen. Neben den Schmelzschneiden und dem Sublimierschneiden ist zum Schneiden des Metallbandes 1 insbesondere das Brennschneiden geeignet. Beim Brennschneiden wird als Schneidgas Sauerstoff eingesetzt, um durch Oxidation eine erodierende Wirkung und damit die Effektivität eines Laserstrahls gegebener Leistung zu erhöhen.

Der Sauerstoff kann durch eine Düse zu dem Brennpunkt, in dem der Laserstrahl auf das Metallband 1 trifft, geblasen werden. Anstelle von Sauerstoff können prinzipiell auch andere Prozessgase oder Gasmischungen verwendet werden. Möglich ist es auch, auf den Einsatz von Prozess- oder Schneidgasen zu verzichten.

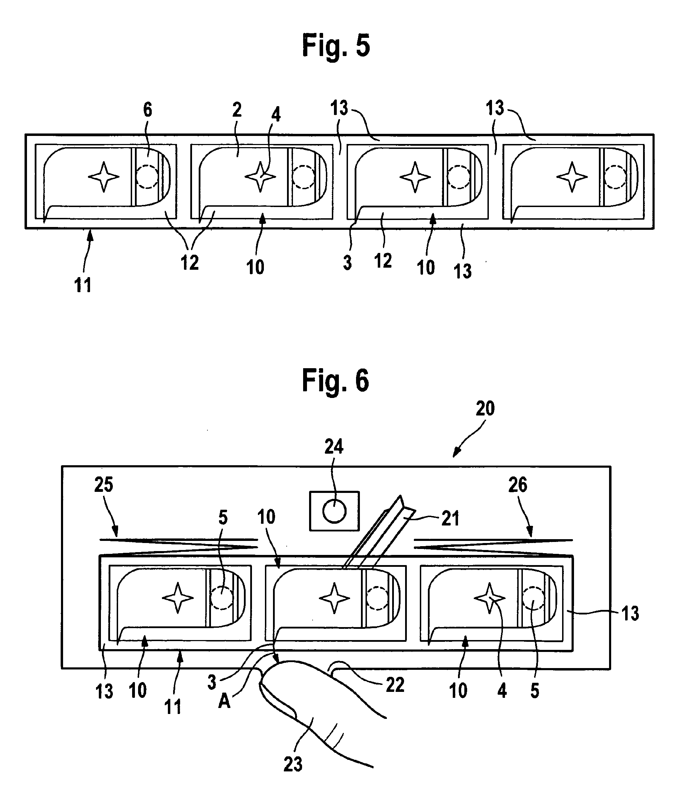

In einem ersten Arbeitsschritt wird aus dem Metallband 1 zunächst nur ein Teil der Kontur der Lanzettenkörper 2 ausgeschnitten, so dass sich das in Figur 2 dargestellte Halbzeugband ergibt. In den Lanzettenkörper 2 werden dabei zwei Ausnehmungen 4, 5 geschnitten. Bei der Ausnehmung 4 handelt es sich um ein Loch mit einer von der Kreisform abweichenden Kontur, die bei dem dargestellten Ausführungsbeispiel sternförmig ist. Die Ausnehmung 4 bildet ein Kupplungselement zum Ankuppeln an einen Lanzettenantrieb eines Stechgeräts. Über das Kupplungselement 4 kann eine Lanzette in eine Drehbewegung versetzt werden. Nähere Details hierzu werden im Folgenden anhand der Figuren 6 bis 9 erläutert.

Bei der Ausnehmung 5 des in Figur 2 gezeigten Lanzettenkörpers 2 handelt es sich um ein kreisförmiges Loch für das Testfeld. Zur Ausbildung des Testfelds wird in einem weiteren Verfahrensschritt auf das Metallband ein Streifen 6 mit Testchemikalien zur Untersuchung einer Körperflüssigkeitsprobe aufgebracht, bevorzugt auflaminiert, beispielsweise aufgeklebt. In Figur 3 ist das in Figur 2 gezeigte Halbzeug mit aufgeklebtem Streifen 6 dargestellt. Bei Kontakt mit einer Körperflüssigkeitsprobe bewirken die Testchemikalien eine Verfärbung des Streifens 6, deren Intensität von der Konzentration des zu bestimmenden Analyten, beispielsweise Glukose, abhängt. Geeignete Testchemikalien zur photometrischen Konzentrationsbestimmungen werden bei handelsüblichen Testelementen, die beispielsweise zur Messung der Glukosekonzentration verwendet werden, eingesetzt und bedürfen daher keiner näheren Erläuterung. Zur Untersuchung einer Körperflüssigkeitsprobe wird diese auf das Testfeld 5 aufgebracht, so dass der Streifen 6 getränkt wird. Der Streifen 6 kann in einem vorbereitenden Verfahrensschritt mittels des Lasers, der auch zum Ausschneiden des Lanzettenkörpers aus dem Metallband 1 verwendet wird, zugeschnitten werden.

Nach dem Aufkleben des Streifens 6 mit den Testchemikalien wird in einem weiteren Verfahrensschritt die restliche Kontur des Lanzettenkörpers 2 ausgeschnitten, so dass sich die in Figur 4 gezeigten Lanzetten 10 ergeben. Für die Schnittqualität ist es dabei günstig, wenn der Streifen 6 mit den Testchemikalien beim Schneiden dem Laserstrahl zugewandt ist, so dass beim Schneiden zuerst der Streifen 6 und danach das in Strahlrichtung darunter liegende Metallblech 1 durchgeschnitten werden.

Der Streifen 6 mit den Testchemikalien enthält Polymerfäden, die beispielsweise ein Vlies, Filz oder Gewebe bilden können. An den Rändern des Streifens 6 werden die Polymerfäden mittels des Lasers verschmolzen und so eine Fusselbildung verhindert.

Bevorzugt wird die Lanzettenspitze 3 in einem weiteren Arbeitsschritt angeschliffen. Dabei wird die Lanzettenspitze 3 abgeflacht. Auf diese Weise kann eine in Fig. 4 dargestellte Kante 7 der Lanzettenspitze 3 als Klinge ausgebildet werden. Beispielsweise kann die Lanzettenspitze 3 und/oder eine Ihrer Kanten angeschrägt werden, wobei sich die Angabe "schräg" in diesem Zusammenhang auf die Ebene des Lanzettenkörpers 2 bezieht. Durch das Anschleifen der Lanzettenspitze 3 lässt sich erreichen, dass die Lanzettenspitze 3 bei einem Einstich leichter in die Hautoberfläche eines Benutzers eindringt und somit der mit einem Einstich verbundene Schmerz reduziert wird.

In einem weiteren Schritt werden die fertigen Lanzetten 10 in Kammern einer in Figur 5 dargestellten Folienverpackung 11 versiegelt. Figur 5 zeigt einen Lanzettenvorratsband mit mehreren Lanzetten 10, die jeweils in Kammern 12 einer Folienverpackung 11 versiegelt sind, wobei die Kammern 12 der Folienverpackung ein Band bilden. Für das in Figur 5 dargestellte Lanzettenvorratsband kann die Folienverpackung 11 beispielsweise als Blisterverpackung ausgebildet werden oder es können zwei aufeinander liegende Folien oder ein in Längsrichtung gefalteter Folienstreifen entlang der in Figur 5 dargestellten Schweißnähte 13 verschweißt werden, um eine luftdichte Versiegelung der auf diese Weise gebildeten Kammern 12 zu bewirken. Besonders gut für die Folienverpackung 11 der Lanzetten 10 sind Kunststofffolien geeignet, insbesondere aus Thermoplasten. Die Schweißnähte 13 können mittels des Lasers erzeugt werden, der auch zum Laserschneiden des Metallbandes 1 eingesetzt wird. Dabei ist es günstig, den Laserstrahl mit einer geeigneten Optik, beispielsweise einer Scanner-Optik, entlang der zu erzeugenden Schweißnähte 13 zu bewegen. Prinzipiell ist es jedoch auch möglich, die in Figur 5 dargestellten Schweißnähte 13 durch Klebeverbindungen zu ersetzen.

Figur 6 zeigt eine schematische Darstellung eines Ausführungsbeispiels eines Stechgeräts 20 zum Erzeugen einer Einstichwunde, in das ein Lanzettenvorratsband gemäß Figur 5 eingesetzt ist. Das in Figur 6 dargestellte Stechgerät 20 zum Erzeugen einer Einstichwunde mittels einer austauschbaren Lanzette 10 umfasst einen Lanzettenantrieb 21 zum Bewegen einer in das Gerät 20 eingesetzten Lanzette 10 für einen Einstich, wobei der Lanzettenantrieb 21 für einen Einstich ein Drehmoment auf eine eingesetzte Lanzette 10 überträgt, so dass diese eine Einstichbewegung in Form einer Drehbewegung ausführt. Der Lanzettenantrieb 21 hat ein Antriebselement in Form einer Welle 21, die eine zu der das Kupplungselement 4 bildenden Ausnehmung passende Form, bei dem dargestellten Ausführungsbeispiel eine Sternform, aufweist und zum Ankuppeln in die Ausnehmung 4 hineingesteckt wird. Das Antriebselement 21 ist zugespitzt, so dass es leicht durch die Folienverpackung 11 hindurch gestochen werden kann. Auf diese Weise läuft die geometrische Achse der Drehbewegung senkrecht zu dem ebenen Lanzettenkörper 2.

Eine Drehbewegung des Antriebselements 21 bewirkt eine Drehbewegung der Lanzette 10 in Richtung des dargestellten Pfeils A um einige Grad (bevorzugt mindestens 15°), so dass die Lanzettenspitze 3 in einem an eine Geräteöffnung 22 angelegten Körperteil 23 eine Einstichwunde erzeugt. Die Lanzettenspitze 3 führt bei einer Einstichbewegung eine Drehbewegung in einer Ebene durch. Die Drehbewegung der Lanzettenspitze 3 ist bogenförmig, insbesondere kreisförmig. Hierfür ist es günstig, wenn das Kupplungselement 4 der Lanzette 10, wie bei dem dargestellten Ausführungsbeispiel, derart angeordnet ist, dass der Schwerpunkt der Lanzette 10 in dem Kupplungselement 4 liegt. Die das Kupplungselement bildende Ausnehmung 4 der Lanzette 10 ist deshalb derart angeordnet, dass sie den physikalischen Schwerpunkt der Lanzette 10 enthält, so dass Unwuchtmomente minimiert werden. Die Lanzettenspitze 3 zerschneidet mit eine Drehbewegung die Folienverpackung 11, so dass die Lanzette 10 bis zu ihrer Verwendung vor schädlichen Umwelteinflüssen geschützt ist. Mit dieser Drehbewegung zum Zerschneiden der Verpackung 11 gelangt die Lanzettenspitze 3 zunächst in eine Vorbereitungsposition (nicht dargestellt). Typischerweise bleibt die Lanzette 10 einige Sekunden in der Vorbereitungsposition in Ruhe, bevor sie für die Einstichbewegung beschleunigt wird, d.h. weitergedreht wird. Auf diese Weise lässt sich vermeiden, dass die Bewegungsgeschwindigkeit bei dem eigentlichen Einstichvorgang durch die Verpackung beeinflusst wird. Prinzipiell ist es aber auch möglich, die Folienverpackung 11 erst bei der Einstichbewegung zu öffnen, also darauf zu verzichten, die Lanzette zunächst in eine Vorbereitungsposition zu drehen und schon dabei die Verpackung zu durchstoßen.

Ein Einstich wird mittels einer schnellen Einstich- und Rückführbewegung ausgeführt. Die Drehrichtung der Lanzette 10 wird mittels des Lanzettenantriebs 21 also umgekehrt, sobald die Lanzettenspitze 3 die vorgegebene Einstichtiefe erreicht hat. Durch eine schnelle Einstich- und Rückführbewegung lässt sich der mit dem Erzeugen eines Einstichs verbundene Schmerz minimieren.

Nach erfolgtem Einstich wird die Lanzettenspitze 3 von der Geräteöffnung 22 weggedreht und das entgegen gesetzte Ende der Lanzette 10, an dem das Testfeld 5 angeordnet ist, aus der Geräteöffnung 22 herausgedreht, so dass die Lanzette in eine Probenaufnahmeposition gelangt und eine aus der erzeugten Einstichwunde gewonnene Körperflüssigkeitsprobe zur Untersuchung auf das Testfeld 5 aufgebracht werden kann. Nach dem Aufbringen einer Körperflüssigkeitsprobe auf das Testfeld 5 wird die Lanzette 10 bei dem dargestellten Ausführungsbeispiel von dem Lanzettenantrieb 21 um etwa 180° gedreht, so dass das Testfeld 5 zu einer in das Stechgerät 20 integrierten Messeinrichtung 24 gelangt, mit der eine Verfärbung des Testfelds 5 photometrisch erfasst und ausgewertet werden kann. Als Alternative zu einem Testfeld mit Testchemikalien, deren Verfärbung photometrisch ausgewertet wird, kann ein Testfeld zur elektrochemischen Konzentrationsbestimmung verwendet werden.

Bei einer anderen Anordnung der Messeinrichtung 24 kann eine Auswertung des Testfeldes 6 auch in der Probenaufnahmeposition erfolgen. Zwar kann die Auswertung einer Messung in der Probenaufaufnahmeposition durch Umwelteinflüsse, insbesondere Streulicht, erschwert werden, jedoch entfällt die Notwendigkeit einer weiteren Drehbewegung der Lanzette, bei der das Testfeld 6 mit einer aufgebrachten Probe durch die Folienverpackung 11 hindurchbewegt werden muss. Bei einer Messung in der Probenaufnahmeposition ist deshalb die Gefahr einer Verschmutzung des Geräteinneren durch Probenflüssigkeit in vorteilhafter Weise reduziert.

Bei dem dargestellten Stechgerät 20 werden unbenutzte Lanzetten 10 des in Figur 5 dargestellten Lanzettenvorratsbands in einem Stapel 25 und benutzte Lanzetten in einem zweiten Stapel 26 gelagert. Die beiden Stapel 25, 26 sind links und rechts von der Geräteöffnung 22 im Geräteinneren angeordnet. Dabei wird die Folienverpackung 11 des in Figur 5 dargestellten Lanzettenvorratsbands jeweils entlang der quer zur Bandrichtung verlaufenen Schweißnähte 13 gefaltet. Ein Vorteil des dargestellten Stechgeräts 20 und des dazugehörenden Lanzettenvorratsbands besteht darin, dass eine Lanzette 10 nach Gebrauch wieder in ihre Ausgangsposition zurück gedreht werden kann, in der sie von der Folienverpackung 11 umgeben ist, so dass gebrauchte Lanzetten 10 hygienisch in dem Stechgerät 20 aufbewahrt werden können und bei einer Entsorgung eines aufgebrauchten Lanzettenvorratsbands die Gefahr von Verletzungen durch Lanzettenspitzen 3 reduziert ist. Bei dem dargestellten Ausführungsbeispiel kann die Lanzette 10 mittels eines einzigen Antriebselements bewegt werden, um die Verpackung 11 zu durchtrennen, die Einstichbewegung auszuführen, die Messposition einzunehmen und nach erfolgter Konzentrationsbestimmung wieder hygienisch sicher in der Verpackung 11 angelegt zu werden.

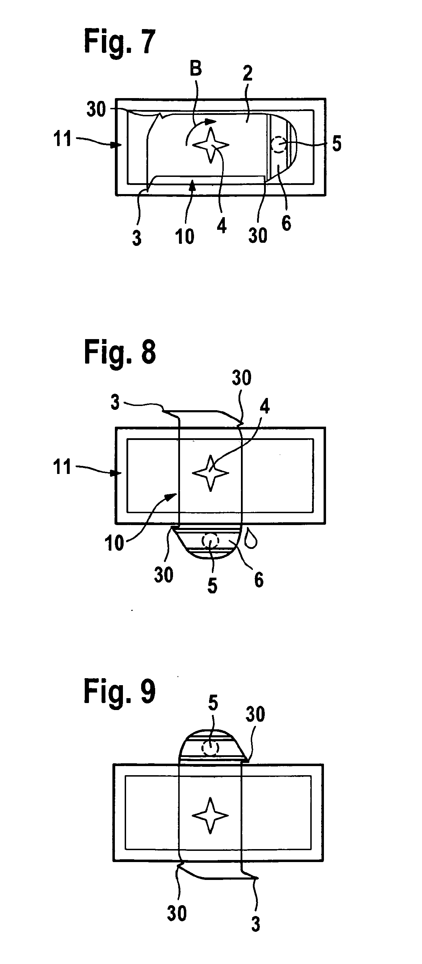

Figur 7 zeigt ein weiteres Ausführungsbeispiel einer Lanzette 10 in einer Folienverpackung 11. Das in Figur 7 dargestellte Ausführungsbeispiel unterschiedet sich von dem im vorhergehenden beschriebenen Ausführungsbeispiel dadurch, dass an dem Lanzettenkörper 2 zusätzlich zu der Lanzettenspitze 3 zum Erzeugen einer Einstichwunde ein Dorn 30, genauer gesagt zwei Dorne 30, zum Aufreißen der die Lanzette 10 umgebenden Folienverpackung 11 angeordnet ist. Zum Aufreißen der Folienverpackung 11 wird die Lanzette 10 zunächst in einer ersten Drehrichtung gedreht, die in Figur 7 durch einen Pfeil B dargestellt ist. Bei dieser ersten Drehbewegung werden die Seiten der Folienverpackung 11 von den beiden Dornen 30 aufgeschlitzt. Anschließend wird mit der Lanzette 10 eine Einstichwunde mit einer Drehbewegung in entgegengesetzter Drehrichtung erzeugt. Bei dem Lanzettenantrieb handelt es sich bevorzugt um einen Federantrieb, dessen Feder bei der ersten Drehbewegung in Richtung des Pfeils B gespannt wird.

Indem die Folienverpackung 11 mittels der Dorne 30 aufgeschnitten wird, kann die darauf folgende Einstichbewegung der Lanzettenspitze 3 ungehindert durchgeführt werden, ohne dass die Lanzettenspitze 3 die Folienverpackung durchstoßen müsste. Dies hat den Vorteil, dass die Einstichbewegung schneller und damit schmerzärmer durchgeführt werden kann. Ferner ist eine Beeinträchtigung der Schärfe der Lanzettenspitze 3, die beim Aufreißen der Folienverpackung 11 auftreten könnte, ausgeschlossen.

Nach erfolgter Einstichbewegung wird die Lanzette 10 in die in Figur 8 dargestellte Position gedreht, in der das Testfeld 5 aus der Geräteöffnung 22 herausragt, so dass eine Körperflüssigkeitsprobe auf das Testfeld 6 aufgebracht werden kann. Mit einer anschließenden Drehung, die bei dem in Figur 6 dargestellten Ausführungsbeispiel eines Stechgeräts 20 180° beträgt, kann das Testfeld 5 in das Innere des Geräts 20 hineingedreht, so dass dessen Verfärbung mittels der Messeinrichtung 24 ausgewertet werden kann. Für das in Figur 6 dargestellte Ausführungsbeispiel eines Stechgeräts 20 ist die Position der Lanzette 10 beim Messen einer Verfärbung des Testfeldes 5 in Figur 9 dargestellt. Nach erfolgter Messung wird die Lanzette 10 wieder in ihre in Figur 7 dargestellte Ausgangsposition zurückgedreht, in der sie bis zur Entsorgung gelagert wird. Wie bereits erwähnt, besteht jedoch auch die Möglichkeit, die Messung in der in Fig. 8 dargestellten Probenaufnahmeposition durchzuführen, so dass die in Fig. 9 dargestellte Position bei einem Ausführungsbeispiel, das in Bezug auf die Anordnung der Messeinrichtung 24 anders als das in Fig. 6 dargestellte Ausführungsbeispiel konstruiert ist, nicht eingenommen werden muss.

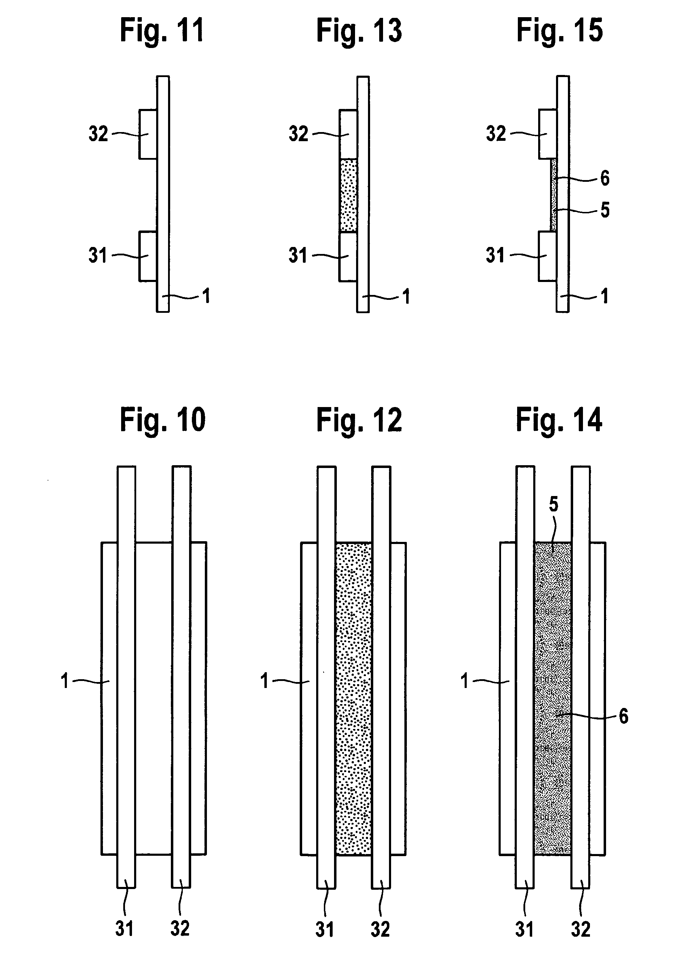

Anstatt ein Streifen 6 mit Testchemikalien zur Ausbildung des Testfelds 5 auf einen Lanzettenkörper 2 oder ein Metallband 1 aufzukleben, kann man auch eine Paste oder Flüssigkeit mit Testchemikalien auf das Metallband 1 oder den Lanzettenkörper 2, der aus dem Metallband 1 ausgeschnitten wird, aufbringen, so dass diese Paste nach Trocknung das Testfeld 5 bildet. Diese alternative Vorgehensweise wird im Folgenden anhand der Figuren 10 bis 15 erläutert.

Figur 10 zeigt schematisch einen Ausschnitt eines Metallbands 1, aus dem wie im Vorhergehenden erläutert, mittels Laserschneiden ein Lanzettenkörper 2 und Lanzettenspitze 3 ausgeschnitten wird. Zur Ausbildung des Testfeldes 5 der auszuschneidenden Lanzetten 10 werden zunächst zwei Begrenzungsstreifen 31, 32 auf das Metallband 1 geklebt. Die Begrenzungsstreifen 31, 32 sind üblicherweise als schmale Kunststoffstreifen ausgebildet, die parallel in einem Abstand von beispielsweise 1 mm bis 3 mm auf den Metallband 1 angeordnet werden. Figur 11 zeigt einen Ausschnitt des Metallbands 1 mit aufgeklebten Begrenzungsstreifen 31, 32. In einem weiteren Verfahrensschritt wird zwischen die beiden Begrenzungsstreifen 31, 32 eine Paste oder Flüssigkeit mit Testchemikalien zur photometrischen Konzentrationsbestimmung aufgebracht. In Figur 12 ist ein Ausschnitt des Metallbands 1 mit aufgeklebten Begrenzungsstreifen 31, 32 und einen mit der Testchemikalien enthaltenden Flüssigkeit oder Paste verfüllten Zwischenraum in einer Draufsicht dargestellt. Figur 13 zeigt eine Querschnittsansicht zu Figur 12.

Anschließend wird die aufgetragene Flüssigkeit oder Paste eingetrocknet, so dass sich zwischen den Begrenzungsstreifen 31, 32 ein Streifen 6 bildet, der das Testfeld 5 enthält. Die Dicke des nach Trocknung zwischen den Begrenzungsstreifen 31, 32 verbleibenden Streifens 6 hängt von dem Feststoffgehalt der aufgetragenen Paste oder Flüssigkeit ab. Figur 15 zeigt in einer Querschnittsansicht die Verhältnisse nach dem Eintrocknen der zwischen den Begrenzungsstreifen 31, 32 aufgebrachten Paste. Die in Figur 15 gezeigte Darstellung ist nicht maßstäblich. Die Dicke der Begrenzungsstreifen beträgt beispielsweise 20 µm, die Dicke des durch Eintrocknung gebildeten Streifens 6 mit Testchemikalien beispielsweise 10 µm und die Dicke des darunter liegenden Metallbands 1 beispielsweise 80 µm. Figur 14 zeigt eine Draufsicht zu Figur 15.

Bei Lanzetten mit einem Testfeld 5, das gemäß dem anhand der Figuren 10 bis 15 beschriebenen Verfahren hergestellt wurde, ist eine Ausnehmung 5, wie sie in Figur 2 dargestellt ist, naturgemäß nicht vorhanden. Die Messeinrichtung 24 zur photometrischen Auswertung einer Farbänderung des Testfelds 5 befindet sich deshalb bei der Auswertung auf derselben Seite des Metallbandes 1 wie das Testfeld 5. Wird dagegen ein Streifen 6 gemäß der anhand der Figuren 1 bis 4 beschriebene Verfahren über eine Ausnehmung 5 in einem Lanzettenkörper 2 geklebt, kann die photometrische Messapparatur wegen der Ausnehmung in dem Lanzettenkörper 2 auf der anderen Seite angeordnet werden, so dass sich der Lanzettenkörper 2 zwischen dem Teststreifen 6 und der Messapparatur befindet.

### Bezugszeichenliste

- 1: Metallband
- 2: Lanzettenkörper
- 3: Lanzettenspitze
- 4: Kupplungselement
- 5: Testfeld
- 6: Streifen mit Testchemikalien
- 7: Lanzettenspitzenkante
- 10: Lanzette
- 11: Folienverpackung
- 12: Kammer der Folienverpackung
- 13: Schweißnähte
- 20: Stechgerät
- 21: Lanzettenantrieb
- 22: Geräteöffnung
- 23: Körperteil
- 24: Messeinrichtung
- 25: Stapel unbenutzter Lanzetten
- 26: Stapel benutzter Lanzetten
- 30: Aufreißdorn
- 31: Begrenzungsstreifen
- 32: Begrenzungsstreifen
- A: Pfeilrichtung für Drehbewegung bei Einstich
- B: Pfeilrichtung für Drehbewegung zum Aufreißen der Folienverpackung

## Patentansprüche

1. Verfahren zum Herstellen einer Lanzette (10), die einen Lanzettenkörper (2) mit einer Lanzettenspitze (3) umfasst, wobei der Lanzettenkörper (2) mitsamt der Lanzettenspitze (3) aus einem Metallband (1) ausgeschnitten wird, **dadurch gekennzeichnet, dass** das Ausschneiden als Laserschneiden erfolgt, das mittels eines Laserstrahls durchgeführt wird.

2. Verfahren nach Anspruch 1, **dadurch gekennzeichnet, dass** beim Schneiden das Metallband (1) relativ zu einem Laserstrahl bewegt wird.

3. Verfahren nach einem der vorhergehenden Ansprüche, **dadurch gekennzeichnet, dass** das Laserschneiden als Brennschneiden vorgenommen wird.

4. Verfahren nach einem der vorhergehenden Ansprüche, **dadurch gekennzeichnet, dass** auf das Metallband (1) ein Streifen (6) mit Testchemikalien zur Untersuchung einer Körperflüssigkeitsprobe aufgebracht wird und mit dem Laser sowohl durch den Streifen (6) als auch durch das anliegende Metallband (1) geschnitten wird.

5. Verfahren nach Anspruch 4, **dadurch gekennzeichnet, dass** beim Laserschneiden zuerst durch den Streifen (6) und anschließend durch das in Strahlrichtung darunter liegende Metallband (1) geschnitten wird.

6. Verfahren nach einem der vorhergehenden Ansprüche, **dadurch gekennzeichnet, dass** der Streifen (6) mit Testchemikalien Polymerfäden enthält, die an Rändern des Streifens (6) mittels des Laserstrahls verschmolzen werden.

7. Verfahren nach einem der vorhergehenden Ansprüche, **dadurch gekennzeichnet, dass** die Lanzettenspitze (3) angeschliffen wird.

8. Verfahren nach einem der vorhergehenden Ansprüche, **dadurch gekennzeichnet, dass** die Lanzette (10) in eine Folienverpackung (11) eingeschweißt wird.

9. Lanzette umfassend einen aus Metallblech ausgeschnittenen Lanzettenkörper (2) mit einer Lanzettenspitze (3), **dadurch gekennzeichnet, dass** das Ausschneiden des Lanzettenkörpers (2) aus dem Metallblech mittels Laserschneiden vorgenommen wurde.

10. Lanzette nach Anspruch 9, **dadurch gekennzeichnet, dass** der Lanzettenkörper (2) zum Ankuppeln an einen Lanzettenantrieb (21) eines Stechgeräts (20) ein Kupplungselement (4) aufweist, über das die Lanzette (10) in eine Drehbewegung versetzbar ist.

11. Lanzette nach Anspruch 10, **dadurch gekennzeichnet, dass** das Kupplungselement eine Ausnehmung (4) mit einer von der Kreisform abweichenden Kontur ist.

12. Lanzette nach einem der Ansprüche 9 bis 11, **dadurch gekennzeichnet, dass** an dem Lanzettenkörper (2) zusätzlich zu der Lanzettenspitze (3) zum Erzeugen einer Einstichwunde ein Dorn (30) zum Aufreißen einer die Lanzette (10) umgebenden Verpackung (11) angeordnet ist.

13. Lanzette nach einem der Ansprüche 9 bis 12, **dadurch gekennzeichnet, dass** der Lanzettenkörper (2) einen auflaminierten Streifen (6) mit Testchemikalien trägt.

14. Lanzette nach einem der Ansprüche 9 bis 13, **dadurch gekennzeichnet, dass** die Lanzettenspitze (3) abgeflacht ist.

15. Lanzettenvorratsband umfassend mehrere Lanzetten (10) nach einem der vorhergehenden Ansprüche 8 bis 14, die jeweils in Kammern (12) einer Folienverpackung (11) versiegelt sind, wobei die Kammern (12) der Folienverpackung (11) ein Band bilden.

16. Stechgerät zum Erzeugen einer Einstichwunde mittels einer austauschbaren Lanzette (10) gemäß der Ansprüche 9 bis 14, umfassend
einen Lanzettenantrieb (21) zum Bewegen einer in das Stechgerät (20) eingesetzten Lanzette (10) für einen Einstich, **dadurch gekennzeichnet, dass** der Lanzettenantrieb (21) für einen Einstich ein Drehmoment auf eine eingesetzte Lanzette (10) überträgt, so dass die eingesetzte Lanzette (10) eine Einstichbewegung in Form einer Drehbewegung ausführt.
